Europäisches Patentamt

European Patent Office

Office européen des brevets

(11) Publication number: **0 173 973**
A2

(12) **EUROPEAN PATENT APPLICATION**

(21) Application number: 85110915.7

(22) Date of filing: 29.08.85

(51) Int. Cl.⁴: **G 01 N 33/577**
G 01 N 33/74, C 12 P 21/00
//(C12P21/00, C12R1:91)

(30) Priority: 29.08.84 JP 181263/84

(43) Date of publication of application:
12.03.86 Bulletin 86/11

(84) Designated Contracting States:
AT BE CH DE FR GB IT LI NL SE

(71) Applicant: Juridical Foundation The
Chemo-Sero-Therapeutic Research Institute
668, Okubo Shimizu-machi
Kumamoto-shi Kumamoto-ken(JP)

(72) Inventor: Nakashima, Toshihiro
7-733-32, Izumi
Kumamoto-shi Kumamoto-ken(JP)

(72) Inventor: Fujita, Haruo
1446-32, Kannabe-machi
Kumamoto-shi Kumamoto-ken(JP)

(72) Inventor: Tsuji, Ichiroh
5-18-27, Wakaba
Kumamoto-shi Kumamoto-ken(JP)

(72) Inventor: Unoki, Masanori
622-6, Shinchi Shimizu-machi
Kumamoto-shi Kumamoto-ken(JP)

(72) Inventor: Eda, Yasuyuki
Yoshino-so 203 2-82, Shimizuhigashi-machi
Kumamoto-shi Kumamoto-ken(JP)

(74) Representative: Vossius Vossius Tauchner Heunemann
Rauh
Siebertstrasse 4 P.O. Box 86 07 67
D-8000 München 86(DE)

(54) **Method for the determination of thyroid-stimulating hormone.**

㊽ Described is a highly sensitive immunoassay method for
determining the amounts of TSH contained in body fluids, in
which an anti–ß–subunit TSH monoclonal antibody having a
specific association constant value is used.

METHOD FOR THE DETERMINATION OF THYROID-STIMULATING

HORMONE

Background of the Invention:

The present invention relates to a method for the determination of thyroid-stimulating hormone (hereinafter

referred to as TSH) based on an immunoassay technique using

monoclonal antibodies.

The determination of the amounts of TSH contained in body fluids, particularly in blood is extensively utilized in diagnosis of such disease as Graves' disease, cetrinism or hypothyroidism, in which the determination is conducted

through immunoassays such as EIA (enzyme immunoassay) or RIA (radioimmunoassay) by using antibodies specific for the constituents of TSH. TSH is constituted of an $\alpha$-subunit and a $\beta$-

subunit. Antigenicity of the $\beta$-subunit of TSH is similar to

antigenicities of the $\beta$-subunits of LH (luteinizing hormone), HCG (human chorigonadotropin) and FSH (follicle-stimulating hormone). Thus, conventional immunoassay techniques (EIA, RIA or the like), in which polyclonal antibodies are used, suffer from poor specificities and reproducibilities, due to the cross reactions between the hormones.

For the purpose of overcoming such disadvantage, new immunoassays have been proposed in which there are used monoclonal antibodies specific for TSH. For example, "Clinical Chemistry, Vol. 28, No.9, pp. 1862 - 1866, 1982" by H. G. Wada et al. discloses an EIA which employs monoclonal

antibodies specific for the $\alpha$-subunit and the $\beta$-subunits of the

above-mentioned hormones.  More specifically, in the disclosed EIA, the anti-α-subunit monoclonal antibodies are used as the solid-phase antibodies (the capture antibodies) for the hormones and the anti-β-subunits monoclonal antibodies are coupled to a peroxidase to be used as the enzyme-labelled antibodies (the enzyme-antibody conjugate).

However, the experiments conducted by the present inventors in the manner as disclosed by Wada et al. proved that the reactivities of the monoclonal antibodies are not necessarily constant with respect to the respective α-subunits and β-subunits and considerably vary depending upon the clones from which such antibodies are prepared.

Immunoassay methodology can be classified generally into two categories:  the two-steps method and the one-step method (the two-site method).  For example, the two-steps EIA includes reacting a sample placed in a tube with a solid-phase adsorbent antibody, washing the reaction product, reacting the washed product with an enzyme-labelled antibody followed by washing, and then determining the amount of the labelled antibody adsorbed onto the solid-phase adsorbent antibody, whereas a sample, a solid-phase adsorbent antibody and a labelled-antibody are simultaneously reacted in the one-step EIA.  Thus, the one-step method is advantageous over the two-steps method in its simplicity because the former requires only one reaction stage and one washing stage while the latter needs the two stages of reaction as well as the two stages of washing.  However, it is generally deemed that the one-step immunoassay is the less sensitive and the less reliable in determining a biological sample. Thus, it is desired, particularly in the one-step immunoassay, to utilize antibodies having high specific reactivites (with a hormone to be determined) as the solid-phase adsorbent antibody and the labelled antibody.

Summary of the Invention:

The primary object of the present invention is to provide an improved immunoassay for the determination of TSH contained in a body fluid, using monoclonal antibodies.

The present inventors have made extensive studies on the development of monoclonal antibodies which make it possible to determine the amount of TSH with high sensitivity, even in the one-step immunoassay method, and also in the two-steps immunoassay as well, and established an effective immuno-assay by using, as the solid-phase adsorbent antibody or as the labelled antibody, monoclonal antibodies specific for the β-subunit of TSH wherein the monoclonal antibodies have a certain association constant (as defined later), i.e. at least $1.0 \times 10^8$ liters/mole.

Thus, according to the present invention, there is provided a method for the determination of TSH by means of an immunoassay, in which a monoclonal antibody specific for the α-subunit of TSH

(anti-α-subunit TSH monoclonal antibody) is used as the solid-phase adsorbent and a monoclonal antibody specific for the β-subunit of TSH (anti-β-subunit TSH monoclonal anti--

body as the labelled antibody or in which an

                                        is used
anti-β-subunit TSH monoclonal antibody/as the solid-phase an                                        is used
adsorbent and/anti-α-subunit TSH monoclonal antibody/as the labelled antibody, the improvement comprising using, as said anti-β-subunit TSH monoclonal antibody, an antibody having the association constant of at least 1.0 x 10 $^8$ liters/mole.

The anti-β-subunit TSH monoclonal antibody to be used in the present invention can be prepared using a
                                              of a mouse
hybridoma obtained by fusion of spleen cells/immunized with TSH

(TSH-whole) or the β-subunit of TSH and myeloma cells. The preparation of monoclonal antibodies is carried out in a conventional manner known to those skilled in the art, as will be

                      the
described below. Among/monoclonal antibodies produced from various clones              anti-β-subunit TSH monoclonal antibodies having the association constant of at least 1.0 x
                are selected
10$^8$ liters/mole/for use in the effective immunoassay of the present invention. The anti-α-subunit TSH monoclonal
            the purpose of
antibody for/the present invention is prepared according to known methods from cellhybrids of spleen cells of a mouse immunized with TSH and myeloma cells.

The features of the present invention will be apparent from the following description, which is only illustrative and not restrictive for the present invention.

Brief Description of the Drawing:

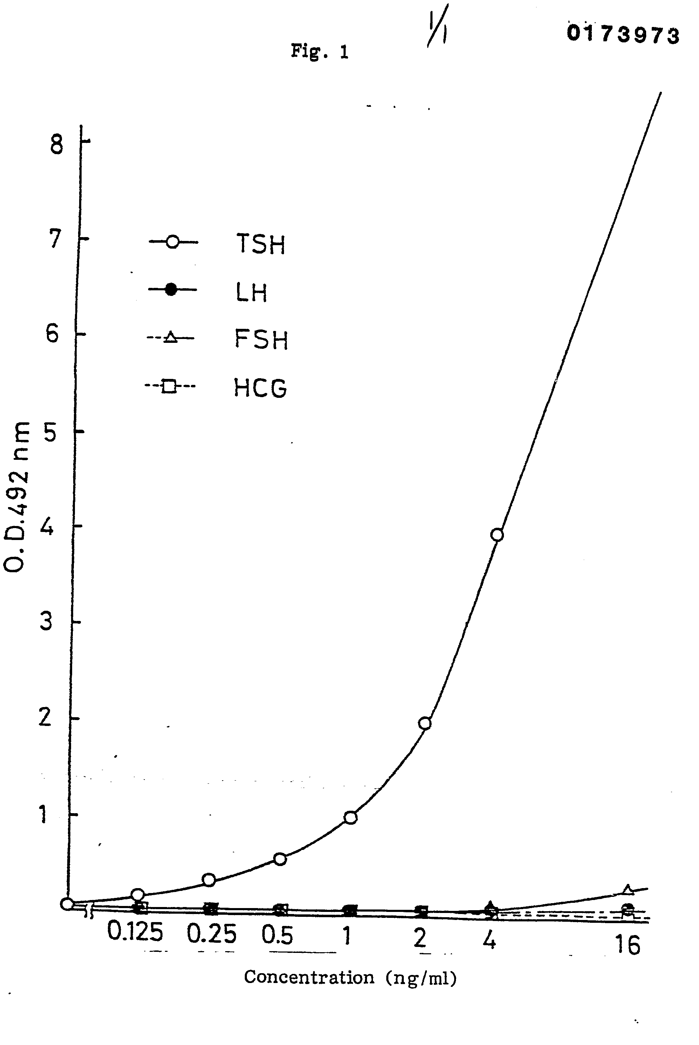

Fig. 1 graphically shows the sensitivity of the assay in accordance with the present invention in determining the hormones.

(A) Preparation of anti-β-subunit monoclonal

antibodies

A W/O suspension for use as antigen for primary immunization is prepared by dissolving 50 μg of TSH (TSH-

whole) or/β-subunit of TSH in 0.9 % physiological saline, and adding to the resultant solution 1.3 ml of FCA (as adjuvant) available from DIFCO Corp..  A booster antigen is prepared by dissolving 50 μg of TSH (TSH-whole) or TSH β-subunit in 0.9 % physiological saline.

Female BALB/c mice, at the ages of four to five weeks, are immunized in accordance with the schedule  -

shown in Table 1.  The immunized mouse spleen cells are fused with mice myeloma cells (e.g. P3-X63-Ag8-U1) by means of the cell fusion technology, for example, as shown in "Hybridoma Process and monoclonal antibodies" edited by T.

Watanabe, pp. 20 - 29, R & D planning Corp. (Japan), 1982, by cloning to obtain seven anti-β-subunit TSH monoclonal

antibodies.

Table 1

| Clone No. | Antigen | Primary Immunization | | Booster Immunization | | | Cell fusion |
|---|---|---|---|---|---|---|---|
| | | Amount | Injecton | Time of Injection | Amount | Injection | |
| 1 | TSH-β | 5μg + FCA | i.p. | 24 days (after primary immunization) | 5 μg | i.v. | Four days after Booster immunization |
| 2 | TSH | do. | do. | 53 days | 10 μg | do. | do. |
| 3 | TSH | do. | do. | 48 days | 5 μg | do. | do. |
| 4 | TSH | do. | do. | do. | do. | do. | do. |
| 5 | TSH | do. | do. | 60 days | 10 μg | do. | do. |
| 6 | TSH | do. | do. | do. | do. | do. | do. |
| 7 | TSH | do. | do. | do. | do. | do. | do. |

- 6 -

The specific reactivities of these monoclonal antibodies were determined with the RIA method by using $^{125}I$-labelled TSH, $^{125}I$-labelled $\alpha$-subunit TSH and $^{125}I$-labelled $\beta$-subunit TSH as the tracers, with the results given in Table 2 in which the values for the specific reactivities are expressed in cpm as compared with the negative controls of the culture supernatants from the mouse myeloma cells (P3-X63-Ag8-U1).

The monoclonal antibodies were also identified with respect to the immunoglobulin-subclass, through Ouchterlony's method, by examining the culture supernatants from the respective clones, by use of antiserum containing antibodies against mouse-$\alpha$, -$\gamma_1$, -$\gamma_{2a}$, -$\gamma_{2b}$, -$\gamma_3$, -$\mu$, -$\kappa$ or -$\lambda$ chain.

The association constants (Ka) of the monoclonal antibodies to TSH were obtained by examining the ascites fluids containing the clones, as followes in the known manner, for example, as described in "Experimental procedures in immunology IX - Determination of association conatantof antibodies" published by Immunological Society of Japan on December 4, 1980, pages 2691 - 2705:

To each of 100 ml diluted ascites fluids containing a monoclonal antibody are added 100 µl of standard antigen (TSH-whole) of a concentration (varying from 0 to 2,000 ng/ml) and 100 µl of $^{125}I$-labelled antigen (TSH-whole) so as to make the competitive reaction at 37 $^{\circ}$C for 2.5 hours. The resultant antigen-antibody product is then subjected to a reaction with anti-mouse immunoglobulin antibody at 37$^{\circ}$C

for 30 minutes, followed by centrifugation to collect the precipitate. The precipitate is determined with respect to radioactivity in cpm. The cpm values are plotted against the concentrations of the standard antigen to form the Scatchard Plots, from which the association constant (Ka) of the subject antibody is calculated.

The results are given in Table 2, demonstrating that, of the anti-$\beta$-subunit TSH monoclonal antibodies obtained, those which have the association constant of at least $1.0 \times 10^8$ liters/mole exhibit particularly high specific activities to $\beta$-subunit TSH.

Table 2

| Clone No. | Specific reactivity | | | | Association constant (liters/mole) | Immunoglobulin subclass | |
|---|---|---|---|---|---|---|---|
| | TSH-whole | TSH $\alpha$ | TSH $\beta$ | Evaluation | | H chain | L chain |
| 1 | 4.1 | 1.4 | 13.7 | $\beta$ | $3.1 \times 10^7$ | $\gamma_1$ | $\kappa$ |
| 2 | 7.0 | 1.4 | 10.8 | $\beta$ | $7.7 \times 10^7$ | $\gamma_1$ | $\kappa$ |
| 3 | 17.6 | 1.3 | 10.6 | $\beta$ | $2.7 \times 10^7$ | $\gamma_1$ | $\kappa$ |
| 4 | 7.0 | 1.6 | 9.6 | $\beta$ | $3.2 \times 10^7$ | $\gamma_1$ | $\kappa$ |
| 5 | 10.3 | 2.2 | 26.4 | $\beta$ | $1.8 \times 10^8$ | $\gamma_1$ | $\kappa$ |
| 6 | 11.1 | 2.2 | 24.6 | $\beta$ | $1.2 \times 10^8$ | $\gamma_1$ | $\kappa$ |
| 7 | 4.5 | 1.9 | 20.0 | $\beta$ | $1.4 \times 10^7$ | $\gamma_2b$ | $\kappa$ |

Example:

The EIA procedure was carried out by using the monoclonal antibodies No.4, No.5 and No.6 as the labelled-antibody for the assay while anti-α-subunit TSH monoclonal antibodies (monoclonal antibodies specific for α-subunit of TSH) are used/as the solid-phase adsorbent.

The anti-α-subunit TSH monoclonal antibodies (hereinafter referred to as α-clones) used were obtained by a conventional method from the fusion of spleen cells from a BALB/c mouse immunized with TSH (TSH-whole) or/the α-subunit of TSH and myeloma cells (P3-X63-Ag8-U1) and had the following characters:

| α-clone | Association constant | Immunoglobulin subclass |
|---------|---------------------|-------------------------|
| (a) | $3.8 \times 10^9$ | $IgG_1$ |
| (b) | $4.5 \times 10^8$ | $IgG_1$ |
| (c) | $3.0 \times 10^8$ | $IgG_1$ |

The α-clones (a), (b) and (c) are described in more detail in the applicant's co-pending application entitled "Method for Determination of Hormones" where the clones designated No.4, No.1 and No.3 in Table 3 in said application correspond the α-clones (a), (b) and (c), respectively. Other α-clones, for example, those described in H.G. Wada et al., can also be used in the present invention.

The two-step EIA was conducted in accordance with the present invention, as follows:

A standard solution (200 µl) having a predetermined concentration of TSH and poly-styrene beads coated with one of the α-clones (the solid-phase) are placed in a tube so as to make the reaction therebetween at 37 °C for 2 hours. The beads were then washed three times with 5 % Tween 20 - PBS. There was added 200 µl of the clone of the anti-β-subunit TSH antibody, No.4, No.5 or No.6 for the reaction at 37 °C for 2 hours, followed by washing three times with 5 % Tween 20 - PBS.

To the washed product was added 500 µl of a solution which has been obtained by dissolving 25 µl of 5 % $H_2O_2$ and 15 mg of o-phenylenediamine·2HCl in 25 ml solution containing 0.1 M citric acid and 0.2 M sodium phosphate, so as to carry out the reaction at 37 °C for one hour in dark. The reaction was terminated by the addtion of 125 µl of 6N $H_2SO_4$.A standard calibration curve for each assay system was obtained by plotting the optical densities (O.D.) of the reaction products (in terms of absorbances at 492 nm) against the concentrations of hormone (TSH). As the sensitivity of each assay system was regarded the concentration value corresponding to the O.D. at the zero concentration multiplied by 2.1. Thus, the value signifies the minimum concentration which can be detected by the assay system.

The results are summarized in Table 3, demonstrating that the use of the monoclonal antibodies Nos. 5 or 6, whose association constants are at least $1.0 \times 10^8$ liters/mole, provide a highly sensitive assay system for

The
TSH. /one-step EIA was also carried out with the substantially same results for the two-steps EIA.

Table 3

Sensitivity of Assay System (in μU/ml)

| Solid phase ＼ Labelled antibody | No.4 | No.5 | No.6 |
|---|---|---|---|
| (a) | 1.0 < | 0.625 | 0.125 |
| (b) | 10.0 < | 1.25 | 0.625 |
| (c) | 20.0 < | 5.0 | 2.5 |

Immunoassay procedures (both the one-step and the two-steps methods) were further conducted for the other hormones (LH, HCG and FSH) in comparison with TSH in a

similar manner as described. Clone No. 6 (the clone with the

st sensitivity in the above) was used as source of the                source of                antibody
ne labelled antibody and/α-clone (a) as /the solid-phase/

ne results are shown in Figure 1, which demonstrates that ne assay system in accordance with the present invention as remarkably high specific reactivity for TSH with substantially no specificities for the other hormones.

Although the present invention has been described with reference to preferred embodiments, it is understood that various changes and modifications may be made by those skilled in the art without departing from the spirit and scope of the invention.

For example, the method of the present invention
the
is not restricted to EIA as described in /Example, but can be applied to the other immunoassays (e.g. RIA) as well.

**0173973**

Claims:

1. A method for the determination of TSH by means of an immunoassay, in which an anti-$\alpha$-subunit TSH monoclonal antibody is used as a solid-phase adsorbent and an anti-ß-subunit TSH monoclonal antibody is used as a labelled antibody or in which an anti-ß-subunit TSH monoclonal antibody is used as the solid-phase adsorbent and an anti-$\alpha$-subunit TSH monoclonal antibody is used as the labelled antibody, characterized in that as anti-ß-subunit TSH monoclonal antibody, an antibody is used having an association constant of at least $1.0 \times 10^{8}$ liters/mole.

2. The method according to claim 1, characterized in that the anti-ß-subunit TSH monoclonal antibody is produced by a hybridoma derived from spleen cells of a mouse immunized with TSH (TSH-whole) or the ß-subunit of TSH fused with myeloma cells.

Fig. 1

1/1

0173973